# EUROPÄISCHE PATENTSCHRIFT

(11) **EP 1 733 778 B1**
(45) Veröffentlichungstag und Bekanntmachung des Hinweises auf die Patenterteilung: **06.06.2012**
(21) Anmeldenummer: 06115260.9
(22) Anmeldetag: 12.06.2006
(51) Int. Cl.: B01D 46/10, B01D 46/52

(54) **Luftfilter**
Air filter
Filtre à air

(30) Priorität: 15.06.2005 DE 202005009468 U
(43) Veröffentlichungstag der Anmeldung: 20.12.2006
(73) Patentinhaber: MANN+HUMMEL GmbH, 71638 Ludwigsburg (DE)
(72) Erfinder: Dirnberger, Timo, 71672, Marbach (DE); Baumann, Christoph, 71706, Markgröningen (DE); Jokschas, Günter, 71540, Murrhardt (DE); Röhrig, Markus, 84036, Landshut (DE)

(56) Entgegenhaltungen:
- WO-A-96/16717
- DE-A1- 19 816 431
- US-A- 5 014 608
- US-A- 5 141 540

## Beschreibung

Die Erfindung betrifft ein Luftfilter für den Ansaugweg eines Verbrennungsmotors, aufweisend ein Filtergehäuse und ein Filterelement, nach der Gattung des Hauptanspruchs.

### Stand der Technik

Es ist beispielsweise aus der DE 198 16 431 A1 ein Filterelement bekannt, bei dem der gefaltete Filtereinsatz an seiner einen Seite eine flächenhaft zumindest teilweise von einer Ebene abweichende Kontur und dabei eine durchgehende Zick-Zack-Faltung aufweist. Das Filterelement kann gemäß der Erfindung zur Optimierung der Filterwirkung durch bessere Raumausnutzung im Bereich dieser Seite des Filtereinsatzes auf einfache Weise an komplexe Strukturen im Ansaugbereich eines Verbrennungsmotors angepasst werden. Durch die gute Anlage des Filterelementes an die Geometrie im Ansaugweg dient die Erfindung auch der Verbesserung des Strömungsverhaltens an dieser Lufteintrittseite des Filterelements.

Die WO96/16717 A1offenbart einen Luftfilter mit einem Filtergehäuse und einem Filtereinsatz. Der Filtereinsatz ist in Halterungen angeordnet und auf dem Filtereinsatz ist anströmseitig ein Gasverteilerelement angeordnet, welches aus einem Fasermedium besteht. Das Gasverteilerelement verhindert schädliche Wirkungen eines direkten Aufpralls des Staubgasstroms. In einer ersten Ausführung ist das Gasverteilerelement direkt auf die Faltenspitzen des Filtereinsatzes aufgeklebt, in einer zweiten Ausführung soll ein direkter Kontakt zwischen den Faltenspitzen und dem Gasverteilerelement verhindert werden. Hierzu ist ein Rahmen vorgesehen, welcher mit dem Filtereinsatz verbunden ist.

Es ist darüber hinaus aus der DE 42 18 396 A1 bekannt, dass Filterelemente, insbesondere für den Einsatz bei Verbrennungsmotoren in Lastkraftwagen und Baumaschinen, mit einer relativ großen Filterfläche hergestellt werden. Diese Filterelemente werden an einer Seite mit einer mindestens am Rand umlaufenden Dichtung versehen um eine Abdichtung der Rohluft- von der Reinluftseite zu erreichen.

Die bekannten Filterelemente können dabei so gestaltet werden, dass bestimmte Geometrien im Filtergehäuse bzw. an den angrenzenden Aggregaten durch eine Zusammenfügung von Einzelfilterelementen mit unterschiedlichen Faltenhöhen berücksichtigt werden. Die Einzelfilterelemente sind dabei jeweils separat hergestellt und über zusätzliche, auch als Scharnier wirkende Verbindungsstege miteinander verbunden.

### Aufgabenstellung

Der Erfindung liegt die Aufgabe zugrunde, einen Luftfilter der eingangs angegebenen Art so auszubilden, dass auf einfache Weise ein optimierbares Strömungsverhalten und eine gute Abdichtung des Filtereinsatzes erreichbar sind.

### Vorteile der Erfindung

Beim erfindungsgemäßen Luftfilter ist in vorteilhafter Weise auf der einen Seite des Filtereinsatzes an den Außenkanten eine umlaufende Dichtung vorhanden, mit der das Filterelement an Gehäuseteilen eines Filtergehäuses anliegt und der Filtereinsatz ist so gestaltet, dass der Bauraum zumindest auf der einen Seite des Filterelements mit minimalem Druckverlust an- oder abströmbar ist.

Der Filtereinsatz ist an- und/oder abströmseitig zumindest teilweise mit einem teildurchlässigen Bauteil abgedeckt, das eine zumindest partielle Vergleichmäßigung der Druckverteilung im Luftstrom bewirkt.

Hierbei ist auf einfache Weise das teildurchlässige Bauteil in einem die gesamte Filteroberfläche bedeckenden Rahmen untergebracht, wobei der restliche Teil des Rahmens der Abstützung auf der Filteroberfläche dient. Das teildurchlässige Bauteil besteht aus einem Lochblech. Mit der Erfindung ist erreicht, dass der Luftstrom in den Luftfilter oder aus dem Luftfilter heraus durch die partielle Abdeckung des Filterelements gleichmäßig verteilt wird.

Die zuvor beschriebene optimale Verteilung des Luftstroms bewirkt, dass dies auch dann erreichbar ist, wenn das Filterelement z.B. mit Staub beladen wird. Ohne die vorgeschlagene Abdeckung würden zunächst nur partielle Bereiche des Filtereinsatzes bestaubt werden und erst danach werden auch die anderen Bereiche bestaubt, was zu einer Änderung des Durchflusses im Bereich eines Luftmassensensors führen kann, der in einem bestimmten Bereich des Filtereinsatzes angebracht ist. Durch die partielle Abdeckung mit dem teildurchlässigen Bauteil ist somit in vorteilhafter Weise eine Signaldrift im Luftmassensensor verhindert oder zumindest stark vermindert.

Bei einer weiteren vorteilhaften Ausführungsform der Erfindung ist das Filterelement im Filtergehäuse über eine umlaufende Dichtung derart gelagert, dass die Dichtung außenliegend in einer Aufnahme des unteren Gehäuseteils liegt und mit dem oberen Gehäuseteil dichtend derart beaufschlagbar ist, dass die Dichtung im wesentlichen mit der Oberfläche des Filtereinsatzes abschließt.

Der erfindungsgemäße Vorschlag zielt auf die Anbringung einer Dichtung, als Dichtschaum, der nicht höher ist als der Filtereinsatz selbst. Mit dieser Dichtung kann bei Luftfilterkonzepten, bei denen oberhalb vom Dichtschaum ein- oder ausgeströmt wird, dieser zusätzliche Bauraum als Ein- oder Ausströmquerschnitt verwendet werden. Hierdurch wird der Druckverlust des Luftfilters, beispielsweise im Ansaugweg eines Verbrennungsmotors, deutlich reduziert.

### Zeichnung

Ausführungsbeispiele des erfindungsgemäßen Filterelements mit einem Filtereinsatz werden anhand der Zeichnung erläutert. Es zeigen:
Figur 1 ein Filterelement nach dem Stand der Technik mit unterschiedlichen Teilfiltereinsätzen,
Figur 2 einen Schnitt durch ein Filtergehäuse mit einem mit der Figur 1 vergleichbaren Filterelement, das jedoch mit einer außenliegenden Dichtung versehen ist und
Figur 3 ein Ausführungsbeispiel eines Filterelements mit einer teildurchlässigen Abdeckung.

### Beschreibung der Ausführungsbeispiele

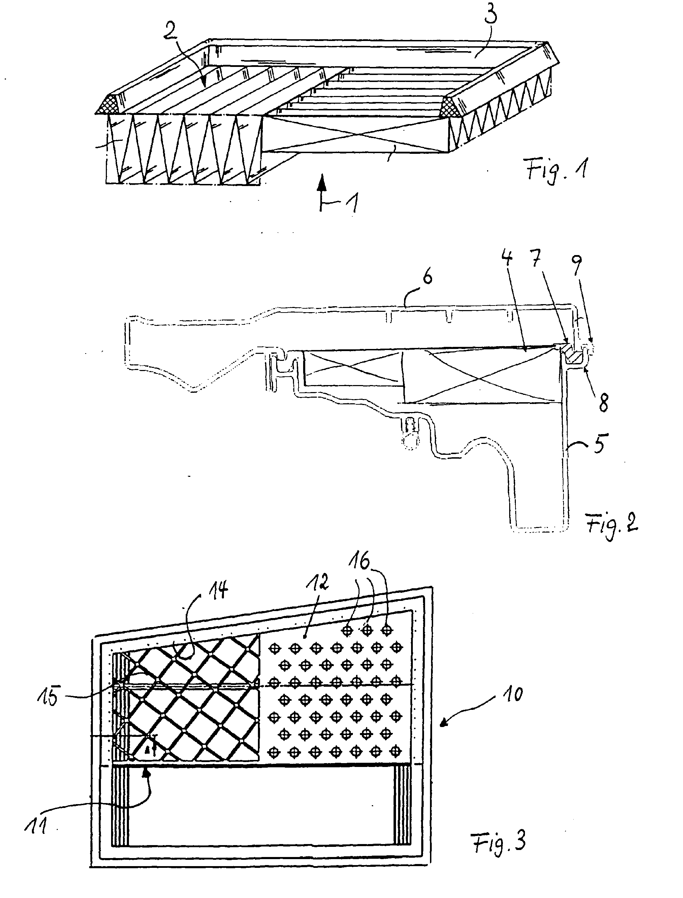

In Figur 1 ist ein Schnitt durch ein Filterelement 1 eines Luftfilters für einen Verbrennungsmotor nach dem Stand der Technik DE 198 16 431 A1 1 gezeigt, das mit einem Filtereinsatz 2 versehen ist, welches im rechten Teil einen Faltenbereich mit einer relativ niedrigen Faltenhöhe und im linken Teil einen Faltenbereich mit einer relativ großen Faltenhöhe aufweist. Das Filterelement 1 ist mit einer umlaufenden Dichtung 3 versehen, die sich im wesentlichen oberhalb der einen Seite des Filterelements 1 befindet und zur Anlage an entsprechende Dichtungsaufnahmen in einem hier nicht dargestellten Filtergehäuse geeignet ist.

Aus Figur 2 ist ein erfindungsgemäßes Filterelement mit einem Filtereinsatz 4 zu entnehmen, der in einem Gehäuseunterteil 5 liegt und über das ein Gehäuseoberteil 6 stülpbar ist. Dieser Filtereinsatz 4 weist eine umlaufende Dichtung 7 auf, die als Dichtschaum im wesentlichen seitlich an den Filtereinsatz 4 angebracht ist und im Gehäuseunterteil 5 in einer Dichtungsaufnahme 8 liegt. Die Dichtwirkung kann dabei durch eine entsprechende Dichtkante 9 am Gehäuseoberteil 6 bewirkt werden, die die Dichtungsaufnahme 8 übergreift und auf die Dichtung 7 drückt. Mit dieser Dichtung 7 kann dann oberhalb vom Dichtschaum ein- oder ausgeströmt und der dadurch entstehende zusätzliche Bauraum als Ein- oder Ausströmquerschnitt verwendet werden, wodurch der Druckverlust des Filterelements mit dem Filtereinsatz 4 reduziert wird.

Bei einem Ausführungsbeispiel nach Figur 3 ist bei einem Filterelement 10 ein Filtereinsatz 11 vorhanden, bei dem an- und/oder abströmseitig zumindest teilweise ein teildurchlässigem Bauteil 12, zum Beispiel ein Lochblech, vorhanden ist, das durch eine zumindest partielle Abdeckung der Filteroberfläche eine Vergleichmäßigung der Druckverteilung und der Partikelbelastung im Luftstrom bewirkt.

Das teildurchlässige Bauteil 12 nach der Figur 3 ist in einem die gesamte Filteroberfläche bedeckenden Rahmen 14 untergebracht sein, wobei der restliche Teil 15 des Rahmens 14 mit einem sehr groben Raster voll durchlässig ist und nur der Abstützung auf der Filteroberfläche dient. Die durch Löcher 16 hervorgerufene Verteilung des Luftstroms bewirkt, dass der Filtereinsatz 11 gleichmäßig zum Beispiel mit dem auszufilternden Staub beladen wird.

## Patentansprüche

1. Luftfilter für den Ansaugweg eines Verbrennungsmotors, aufweisend ein Filtergehäuse und ein Filterelement, wobei das Filtergehäuse über ein Gehäuseoberteil (6) und ein Gehäuseunterteil (5) verfügt und wobei das Filterelement aus mindestens einem Filtereinsatz (4, 11) und einer auf der einen Seite des Filtereinsatzes (4, 11) an den Außenkanten umlaufenden Dichtung (7), mit der das Filterelement (4, 11) an den Gehäuseteilen (5, 6) des Filtergehäuses anlegbar ist, besteht, wobei der Filtereinsatz (4, 11) so gestaltet ist, dass die umlaufende Dichtung (7) als Dichtschaum ausgeführt ist und im wesentlichen seitlich an den Filtereinsatz (4, 11) angebracht ist und im Gehäuseunterteil (5) in einer Dichtungsaufnahme (8) liegt, wobei der oberhalb des Dichtschaums entstehende zusätzliche Bauraum als Ein- oder Ausströmquerschnitt verwendbar ist, und der Bauraum auf der einen Seite des Filterelements mit minimalem Druckverlust an- oder abströmbar ist, **dadurch gekennzeichnet, dass** der Filtereinsatz (11) an- und/oder abströmseitig zumindest teilweise mit einem teildurchlässigem Bauteil (12) abgedeckt ist, wobei das teildurchlässige Bauteil als Lochblech ausgebildet ist, das eine zumindest partielle Vergleichmäßigung der Druckverteilung im Luftstrom bewirkt, wobei das teildurchlässige Bauteil (12) in einem die gesamte Filteroberfläche bedeckenden Rahmen (14) untergebracht ist, wobei der restliche Teil (15) des Rahmens(14) mit einem sehr groben Raster voll durchlässig ist und der Abstützung auf der Filteroberfläche dient.

2. Luftfilter nach Anspruch 1, **dadurch gekennzeichnet, dass**
der Filtereinsatz im Filtergehäuse über die umlaufende Dichtung (7) derart gelagert ist, dass die Dichtung (7) außenliegend in einer Aufnahme (8) des unteren Gehäuseteils (5) liegt und mit dem oberen Gehäuseteil (6) dichtend derart beaufschlagbar ist, dass die Dichtung (7) im wesentlichen mit der Oberfläche des Filtereinsatzes (4) abschließt.

## Claims

1. Air filter for the intake tract of an internal combustion engine, featuring a filter housing and a filter element, wherein the filter housing has a housing upper part (6) and a housing lower part (5) and wherein the filter element consists of at least one filter insert (4, 11) and a circumferential sealing (7) at one side of the filter insert (4, 11) around the outer edges with which the filter insert (4, 11) can be placed against the housing components (5, 6) of the filter housing, wherein the filter insert (4, 11) is designed in such a way that the circumferential sealing (7) is designed as sealing foam and substantially attached laterally at the filter insert (4, 11) and placed in the housing lower part (5) an a seal fixing (8), wherein the additional space generated above the sealing foam can be used as intake or discharge cross-section, and the space on one side of the filter element can be flowed through or flowed out with a minimum of pressure loss, **characterized in that** the filter insert (11) is covered at least partially with a partially permeable component (12) at the on-flow and/or off-flow side, wherein the partially permeable component is designed as perforated plate which causes an at least partial homogenization of the pressure distribution in the air flow, wherein the partially permeable component (12) is placed in a frame (14) covering the whole filter surface, wherein the remaining part (15) of the frame (14) is totally permeable with a very coarse screen and serves as a support on the filter surface.

2. Air filter according to claim 1, **characterized in that** the filter insert in the filter housing is supported above the circumferential sealing (7) in such a way that the sealing (7) is placed outside in a support (8) of the housing lower part (5) and sealingly applied to the housing upper part in such a way that the sealing (7) substantially closes with the surface of the filter insert (4).

## Revendications

1. Filtre à air pour le trajet d'aspiration d'un moteur à combustion interne, présentant un boîtier de filtre et un élément filtrant, le boîtier de filtre disposant d'une partie supérieure de boîtier (6) et d'une partie inférieure de boîtier (5) et l'élément filtrant étant composé d'au moins un insert de filtre (4, 11) et d'un joint (7) entourant les bords extérieurs d'un côté de l'insert de filtre (4, 11), joint au moyen duquel l'insert de filtre (4, 11) peut être appliqué contre les parties (5, 6) du boîtier de filtre, l'insert de filtre (4, 11) étant conçu de telle manière que le joint périphérique (7) est réalisé en tant que mousse d'étanchéité, monté essentiellement sur le côté de l'insert de filtre (4, 11) et placé dans la partie inférieure de boîtier (5) dans un logement pour joint (8), l'espace supplémentaire généré au-dessus de la mousse d'étanchéité pouvant être utilisé comme section d'entrée ou de sortie et l'espace situé d'un côté de l'élément filtrant pouvant être traversé, avec une perte minime de pression, par un flux d'entrée ou de sortie, **caractérisé en ce que** l'insert de filtre (11) est recouvert, du côté entrée et/ou sortie, au moins en partie par un composant (12) partiellement perméable, le composant partiellement perméable étant réalisé en tant que tôle perforée provoquant une homogénéisation au moins partielle de la répartition de la pression dans le flux d'air, le composant (12) partiellement perméable étant logé dans un cadre (14) couvrant l'intégralité de la surface du filtre, la partie restante (15) du cadre (14) avec un quadrillage très grossier étant entièrement perméable et servant d'appui sur la surface du filtre.

2. Filtre à air selon la revendication 1, **caractérisé en ce que** l'insert de filtre dans le boîtier de filtre au-dessus du joint périphérique (7) est logé de telle manière que le joint (7) est placé à l'extérieur dans un logement (8) de la partie inférieure de boîtier (5) et peut être appliqué de manière étanche à la partie supérieure de boîtier (6) de sorte que le joint (7) se termine essentiellement au niveau de la surface de l'insert de filtre (4).
